# EUROPÄISCHE PATENTSCHRIFT

(11) **EP 3 448 271 B1**
(45) Veröffentlichungstag und Bekanntmachung des Hinweises auf die Patenterteilung: **03.01.2024**
(21) Anmeldenummer: 17719539.3
(22) Anmeldetag: 19.04.2017
(51) Int. Cl.: A61B 17/122

(54) **ÜBERSETZTER ANEURYSMEN-CLIP**
CONVERTED ANEURISM CLIP
CLAMP DÉMULTIPLIÉ POUR ANÉVRISME

(30) Priorität: 25.04.2016 DE 102016107587
(43) Veröffentlichungstag der Anmeldung: 06.03.2019
(73) Patentinhaber: Aesculap AG, 78532 Tuttlingen (DE)
(72) Erfinder: SAUTER, Wolfgang, 78603 Renquishausen (DE)
(74) Vertreter: Winter, Brandl - Partnerschaft mbB
(86) Internationale Anmeldenummer: PCT/EP2017/059292
(87) Internationale Veröffentlichungsnummer: WO 2017/186542

(56) Entgegenhaltungen:
- WO-A1-2014/001008
- WO-A1-2014/012718
- DE-A1-102014 114 946
- US-A- 3 326 217

## Beschreibung

Die vorliegende Erfindung betrifft einen chirurgischen Clip, insbesondere Aneurysmen-Clip, mit zwei Clipbranchen, welche an ihren proximalen Enden über ein erstes Federelement, insbesondere eine Schenkelfeder, federelastisch verbunden und vorgespannt sind, welche um einen von dem ersten Federelement ausgebildeten Federdrehpunkt drehbar sind und welche an ihren distalen, freien Enden in einer Ruhestellung des chirurgischen Clips durch die Schließkraft des ersten Federelements parallel aneinander anliegend gehalten sind.

Derartige chirurgische Clips, welche zwei Clipbranchen bzw. Maulteilbranchen aufweisen, an ihren proximalen Enden über ein federelastisches Element miteinander verbunden sind und an ihren distalen, freien Enden in einer Ruhestellung des Clips mit einer vorgegebenen Schließkraft parallel aneinander anliegend gehalten sind, sind aus dem Stand der Technik bekannt. Es sei angeführt, dass in der vorliegenden Anmeldung die Begriffe "proximal" und "distal" mit Bezug auf einen Operateur/ Arzt/ Anwender zu verstehen sind und "proximal" somit die zum Anwender hin gerichtete Richtung und "distal" die vom Anwender weg bzw. die zum Patienten hin gerichtete Richtung bezeichnen. Die bekannten chirurgischen Clips bzw. Aneurysmen-Clips verwenden dabei eine Schenkelfeder als federelastisches Element und weisen vorzugsweise Überkreuzungsabschnitte zwischen ihren proximalen und distalen Enden auf, an welchen sich die Clipbranchen gegenseitig überkreuzen. Eine Druckkrafteinleitung zum Öffnen der Clipbranchen erfolgt insbesondere auf einem zwischen den Überkreuzungsabschnitten und der Schenkelfeder angeordneten Krafteinleitungspfad.

Wesentlich bei derartigen chirurgischen Clips ist ihre Schließkraft, welche präzise eingestellt sein muss und bei einer Verwendung des chirurgischen Clips als Implantat auch auf Dauer gegeben sein muss. Derartige chirurgische Clips werden nämlich eingesetzt, um ein Aneurysma, i.e. eine Arterienerweiterung bzw. eine arterielle Aussackung, worunter eine spindel- oder sackförmige, lokalisierte und permanente Erweiterung eines Blutgefäßquerschnitts zu verstehen ist, abzuklemmen und somit das Aneurysma von dem Blutkreislauf auszuschließen. Chirurgische Clips bzw. Aneurysmen-Clips müssen dabei kompakt bzw. platzsparend ausgebildet sein, da in der Chirurgie Blutgefäße bzw. Aneurysmen und dergleichen oft in räumlich sehr beengter Umgebung abgeklemmt werden müssen.

Um einen derartigen chirurgischen Clip bzw. Aneurysmen-Clip zu öffnen, werden die proximal von dem Überkreuzungsabschnitt gelegenen Enden der Clipbranchen mit einem Werkzeug, beispielsweise einer Clipanlegezange gefasst und gegen die Federkraft der Schenkelfeder einander angenähert. Dabei verhalten sich eine Kraft bzw. eine Belastung, welche über das Werkzeug auf die Feder aufgebracht wird, und eine Öffnungsweite bzw. ein Öffnungswinkel der distalen Enden der Clipbranchen zueinander proportional. Mit anderen Worten steigt mit einem zunehmenden Öffnungswinkel die Federkraft kontinuierlich an. Aufgrund der hohen erforderlichen Schließkraft der Schenkelfeder sowie der kompakten Bauweise des Aneurysmen-Clips ist ein Arbeitsbereich bzw. eine Öffnungsweite der Schenkelfeder in den aus dem Stand der Technik bekannten chirurgischen Clips bzw. Aneurysmen-Clips begrenzt. Der erreichbare Öffnungswinkel bzw. die erreichbare Öffnungsweite ist in anderen Worten im Stand der Technik aufgrund der erforderlichen Schließkraft und der erforderlichen kompakten Bauweise des Clips limitiert.

Die DE 10 2014 114 946 A1 offenbart in einer Ausführungsform einen chirurgischen Clip gemäß der Präambel des Anspruchs 1. In einer weiteren Ausführungsform offenbart sie einen chirurgischen Clip, dessen Klemmbranchen über eine Bügelfeder zusammengehalten werden. Die Klemmbranchen weisen dabei an ihren proximalen Enden zueinander weisende Abrollflächen auf, an denen die Klemmbranchen abrollen, wenn die freien Enden der Klemmbranchen geöffnet werden. An zumindest einer Abrollfläche ist dabei ein nockenförmiger Abschnitt vorgesehen, welcher bewirkt, dass sich beim Öffnen bzw. Schließen der Klemmbranchen der Drehpunkt der beiden Klemmbranchen verändert und der Gradient der Klemmkraft mit zunehmendem Öffnungswinkel abnimmt.

Die Nachteile des Standes der Technik sollen durch die vorliegende Erfindung vermieden bzw. wenigstens vermindert werden. Insbesondere soll ein chirurgischer Clip bzw. Aneurysmen-Clip bereitgestellt werden, welcher bei der bisher erreichten Schließkraft eine wesentlich größere Öffnungsweite an den distalen Enden der Clipbranchen bzw. Maulteilbranchen bietet, ohne die Geometrie bzw. die äußeren Abmessungen des chirurgischen Clips zu vergrößern.

Diese Aufgabe wird insbesondere durch einen chirurgischen Clip mit den Merkmalen des Anspruchs 1 gelöst. Vorteilhafte Ausführungsformen und Weiterbildungen sind Teil der Unteransprüche.

Die Erfindung betrifft einen chirurgischen Clip, insbesondere Aneurysmen-Clip, mit zwei Clipbranchen/ Maulteilbranchen, welche an ihren proximalen Enden über ein erstes Federelement, insbesondere eine Schenkelfeder, federelastisch verbunden und vorgespannt sind, welche um einen von dem ersten Federelement ausgebildeten Federdrehpunkt/ um eine von dem ersten Federelement ausgebildete Federdrehachse drehbar sind und welche an ihren distalen, freien Enden in einer Ruhestellung des chirurgischen Clips durch die Schließkraft des ersten Federelements parallel aneinander anliegend gehalten sind, wobei der chirurgische Clip eine Übersetzungseinrichtung aufweist, welche ab einem vorbestimmten Öffnungswinkel der distalen, freien Enden der beiden Clipbranchen ein Verhältnis von Öffnungskraft, welche zum Öffnen der Clipbranchen an einem Krafteinleitungspfad aufgebracht wird, zu Öffnungswinkel verringert. Zumindest eine der Clipbranchen weist einen zusätzlichen Drehpunkt/ eine zusätzliche Drehachse auf, welcher/ welche distal zu dem Federdrehpunkt und proximal zu dem Krafteinleitungspfad angeordnet ist und um welchen/ um welche die zumindest eine Clipbranche unter Überwindung einer federelastischen Vorspannung zumindest eines zweiten Federelements, insbesondere einer Blattfeder oder einer Biegefeder, drehbar ist.

Der vorbestimmte Öffnungswinkel ist vorzugsweise sehr gering und liegt in einem Bereich zwischen 0° und 20°, weiter vorzugsweise zwischen 0° und 10°. In vorteilhafter Weise weisen die beiden Clipbranchen Überkreuzungsabschnitte zwischen ihren proximalen und distalen Enden auf, an welchen sich die Clipbranchen gegenseitig überkreuzen, wobei die Öffnungskraft, insbesondere Druckkraft, zum Öffnen der Clipbranchen auf dem zwischen den Überkreuzungsabschnitten und dem ersten Federelement angeordneten Krafteinleitungspfad erfolgt.

Durch einen derartigen chirurgischen Clip wird zum einen erreicht, dass die erforderliche Schließkraft in der Ruhestellung des Clips vorzugsweise ausschließlich über das erste Federelement, welches erfindungsgemäß beispielsweise eine Schenkelfeder ist, aufgebracht wird und dadurch die freien distalen Enden des Clips parallel aneinander anliegend gehalten werden. Es wird somit die benötigte Schließkraft zum Schließen des Clips bereitgestellt. Diese Schließkraft muss erfindungsgemäß jedoch zum Öffnen des chirurgischen Clips nicht oder nur teilweise überwunden werden. Durch die Übersetzungseinrichtung der vorliegenden Erfindung wird nämlich erreicht, dass ab einem vorbestimmten Öffnungswinkel eine zum Öffnen des chirurgischen Clips aufzubringende Kraft - im Vergleich zur Schließkraft des Clips - vermindert bzw. reduziert wird und/oder eine an dem Krafteinleitungspfad aufgebrachte Kraft zum Öffnen der Clipbranchen in eine größere Öffnungsweite bzw. einen größeren Öffnungswinkel übersetzt wird.

Gemäß der vorliegenden Erfindung ist es vorgesehen, zum Öffnen des chirurgischen Clips eine federelastische Vorspannung zumindest eines zweiten Federelements, welches vorzugsweise eine Blattfeder oder eine Biegefeder ist, zu überwinden. Das zweite Federelement bzw. dessen Vorspannung in Schließrichtung des Clips ist derart ausgebildet, dass im Vergleich zu dem die Schließkraft erzeugenden ersten Federelement eine geringere Federkraft überwunden werden muss, so dass sich die zumindest eine Clipbranche des chirurgischen Clips einfacher öffnen lässt. Erfindungsgemäß dreht sich diese Clipbranche dann beim Öffnen des Clips unter Auslenkung/ Verbiegung des zweiten Federelements um einen zusätzlichen Drehpunkt/ eine zusätzliche Drehachse, welcher/ welche zwischen dem Federdrehpunkt und dem Krafteinleitungspfad angeordnet ist. Der zusätzliche Drehpunkt befindet sich somit näher an dem Krafteinleitungspfad als der Federdrehpunkt des ersten Federelements. Durch diese Verlagerung bzw. Verschiebung des Drehpunkts beim Öffnen der zumindest einen Clipbranche näher zum Krafteinleitungspfad hin wird somit in vorteilhafter Weise eine übersetzte Öffnungsweite bzw. ein übersetzter Öffnungswinkels der zumindest einen Clipbranche erreicht.

In anderen Worten führt eine Auslenkung der zumindest einen Clipbranche an dem Krafteinleitungspfad aufgrund des zusätzlichen, näher an dem Krafteinleitungspfad gelegenen Drehpunkts zu einer größeren erreichbaren Öffnungsweite bzw. einem größeren erreichbaren Öffnungswinkel des chirurgischen Clips. Es wird somit erfindungsgemäß bei einer gegebenen Schließkraft eine wesentlich größere Öffnungsweite von zumindest einer Clipbranche an den distalen Enden der Clipbranchen bzw. Maulteilbranchen erreicht, ohne die Geometrie und die äußeren Abmessungen des chirurgischen Clips zu vergrößern. Durch das Vorsehen des zweiten Federelements wird insbesondere die aufzubringende Kraft zum Öffnen des chirurgischen Clips ab dem erfindungsgemäß vorgesehenen vorbestimmten Öffnungswinkel reduziert. Durch das Vorsehen eines näher an dem Krafteinleitungspfad gelegenen zusätzlichen Drehpunkts von zumindest einer Clipbranche kann eine übersetzte Öffnungsweite bzw. ein übersetzter Öffnungswinkel der zumindest einen Clipbranche realisiert werden.

Ein vorteilhaftes Ausführungsbeispiel ist dadurch gekennzeichnet, dass die beiden Clipbranchen jeweils einen zusätzlichen Drehpunkt aufweisen, um welche die beiden Clipbranchen jeweils unter Überwindung einer federelastischen Vorspannung eines zweiten und eines dritten Federelements, welche insbesondere jeweils als eine Blattfeder oder eine Biegefeder ausgebildet sind, drehbar sind, wobei die beiden zusätzlichen Drehpunkte vorzugsweise von dem ersten Federelement distal und von dem Krafteinleitungspfad proximal gleich beabstandet sind.

Es ist somit gemäß dieser Ausführungsform an der einen Clipbranche ein erster zusätzlicher Drehpunkt und ein zweites Federelement, sowie an der anderen Clipbranche ein zweiter zusätzlicher Drehpunkt und ein drittes Federelement vorgesehen. Somit weisen nach dieser Ausführungsform zwingend beide Clipbranchen jeweils die voranstehend beschriebene Übersetzungseinrichtung auf. Dadurch sind nach dieser Ausführungsform beide Clipbranchen in größerem Maße auslenkbar und die zu erreichende Öffnungsweite bzw. der zu erreichende Öffnungswinkel werden somit vergrößert. In vorteilhafter Weise liegt eine gleiche Beabstandung der beiden zusätzlichen Drehpunkte jeweils von dem ersten Federelement distal und dem Krafteinleitungspfad proximal vor, so dass eine symmetrische Auslenkung der beiden Clipbranchen beim Öffnen des chirurgischen Clips gewährleistet werden kann. In anderen Worten ist somit der Abstand des ersten zusätzlichen Drehpunkts von dem ersten Federelement an der einen Clipbranche gleich dem Abstand des zweiten zusätzlichen Drehpunkts von dem ersten Federelement an der anderen Clipbranche.

Es ist vorteilhaft, wenn der chirurgische Clip eine Federwegbegrenzungseinrichtung, insbesondere einen Anschlag, aufweist, welche eine Auslenkung und/oder einen Öffnungswinkel der beiden Clipbranchen um den von dem ersten Federelement ausgebildeten Federdrehpunkt begrenzt. Insbesondere wird durch eine derartige Federwegbegrenzungseinrichtung/ einen derartigen Anschlag der erfindungsgemäße, vorbestimmte Öffnungswinkel bzw. die vorbestimmte Öffnungsweite der distalen Enden der beiden Clipbranchen definiert.

Erfindungsgemäß kann es somit vorgesehen sein, dass die Schließkraft des ersten Federelements zunächst teilweise überwunden werden muss und eine Drehung der beiden Clipbranchen um den von dem ersten Federelement ausgebildeten Federdrehpunkt erfolgt. Diese teilweise Überwindung der Schließkraft des ersten Federelements erfolgt bis zu einem vorbestimmten Punkt bzw. Anschlag bzw. Öffnungswinkel, welcher durch die Federwegbegrenzungseinrichtung definiert ist. Der Punkt bzw. Anschlag wird dabei erfindungsgemäß derart festgelegt, dass die Kraft, welche für die Drehung der Clipbranchen um den Federdrehpunkt nötig ist, nicht zu groß wird. Durch den Anschlag wird die Drehung der Clipbranchen um den Federdrehpunkt begrenzt, so dass bei einer weiteren Druckkraftaufbringung keine weitere Auslenkung der distalen Enden der Clipbranchen um den Federdrehpunkt mehr möglich ist.

Die erfindungsgemäße Übersetzungseinrichtung ist somit gekennzeichnet durch eine Federwegbegrenzungseinrichtung zum Begrenzen einer Auslenkung um den von dem ersten Federelement ausgebildeten Federdrehpunkt und eine Drehpunktwechseleinrichtung, welche nach dem Begrenzen der Auslenkung der Clipbranchen um den Federdrehpunkt einen Wechsel des Drehpunkts der Clipbranchen näher zu dem Krafteinleitungspfad hin vornimmt und eine Drehung um den neuen Drehpunkt durch eine Verbiegung des zweiten und/oder dritten Federelements ermöglicht.

Insbesondere ist es dabei von Vorteil, wenn bei einer Kraftaufbringung an dem Krafteinleitungspfad zunächst eine Drehung der Clipbranchen um den Federdrehpunkt und anschließend erst eine Drehung der Clipbranchen um den zumindest einen/ die beiden zusätzlichen Drehpunkte unter Auslenkung des zweiten Federelements/ des zweiten und des dritten Federelements erfolgt. Dadurch wird erreicht, dass in einer Schließstellung des chirurgischen Clips die Schließkraft in jedem Fall nur durch das erste Federelement aufgebracht wird. Es wird somit ausgeschlossen, dass das zweite Federelement/ die zweiten und dritten Federelemente die Schließkraft des ersten Federelements beeinflussen/ beeinträchtigen/ reduzieren.

Ein vorteilhaftes Ausführungsbeispiel der vorliegenden Erfindung ist dadurch gekennzeichnet, dass die zwei Clipbranchen an ihren Überkreuzungsabschnitten jeweils einen Federabschnitt und einen Verbindungsabschnitt aufweisen, wobei der Federabschnitt der einen Clipbranche das zweite Federelement und der Federabschnitt der anderen Clipbranche das dritte Federelement aufweisen und wobei die Federabschnitte jeweils mit dem ersten Federelement verbunden sind, wobei die Verbindungsabschnitte der zwei Clipbranchen derart drehbar miteinander verbunden sind, dass die zusätzlichen Drehpunkte der beiden Clipbranchen als ein gemeinsamer zusätzlicher Drehpunkt ausgebildet sind und die Clipbranchen jeweils um den gemeinsamen zusätzlichen Drehpunkt über eine Auslenkung und/oder Verbiegung und/oder Überwindung der federelastischen Vorspannung der zweiten und dritten Federelemente drehbar sind.

Gemäß diesem Ausführungsbeispiel ist es somit vorgesehen, dass sich die beiden Clipbranchen jeweils an ihren Überkreuzungsabschnitten zu dem proximalen Ende hin in zwei Abschnitte aufspalten. Ein Abschnitt bzw. der Federabschnitt ist dabei jeweils mit dem ersten Federelement bzw. der Schenkelfeder verbunden und weist das zweite bzw. das dritte Federelement auf. Die Federabschnitte sind dabei jeweils außenliegend an den beiden Clipbranchen vorgesehen, so dass eine Druckkrafteinleitung zum Öffnen des chirurgischen Clips an den Federabschnitten erfolgt. Die anderen Abschnitte bzw. die Verbindungsabschnitte der beiden Clipbranchen sind miteinander verbunden bzw. gekoppelt und bilden den gemeinsamen zusätzlichen Drehpunkt aus. Der Drehpunkt liegt insbesondere in Längsrichtung gesehen auf einer Mittelachse des chirurgischen Clips bzw. auf einer von den Anlageflächen der beiden Clipbranchen in geschlossenem Zustand des Clips aufgespannten Ebene. Die Verbindungsabschnitte sind somit innenliegend an den beiden Clipbranchen vorgesehen. Es versteht sich von selbst, dass der zusätzliche Drehpunkt gemäß dem Kerngedanken der vorliegenden Erfindung wiederum proximal von dem Krafteinleitungspfad und distal von dem ersten Federelement bzw. der Schenkelfeder angeordnet ist. Die Clipbranchen sind gemäß diesem Ausführungsbeispiel zum einen über das erste Federelement bzw. die Schenkelfeder miteinander verbunden, zum anderen sind die Clipbranchen auch an dem zusätzlichen gemeinsamen Drehpunkt miteinander verbunden. Es wird somit ein zusätzlicher gemeinsamer Drehpunkt bereitgestellt, um welchen die beiden Clipbranchen jeweils drehbar sind. Gemäß dieser Ausführungsform wird die aufzubringende Kraft insbesondere durch die Federabschnitte der beiden Clipbranchen, welche die zweiten bzw. dritten Federelemente enthalten, reduziert und die übersetzte Öffnungsweite wird insbesondere durch den gemeinsamen zusätzlichen Drehpunkt, welcher an den Verbindungsabschnitten ausgebildet ist, erreicht. Wird eine Druckkraft zum Öffnen der Clipbranchen auf die Federabschnitte eingeleitet, erfolgt eine Auslenkung/ Verbiegung der jeweils an den Federabschnitten vorgesehenen zweiten und dritten Federelemente und eine damit einhergehende Drehung der beiden Clipbranchen, insbesondere der Verbindungsabschnitte und der distalen Enden um den gemeinsamen zusätzlichen Drehpunkt.

Dabei ist es vorteilhaft, wenn die Verbindungsabschnitte der beiden Clipbranchen über eine Bolzenverbindung drehbar miteinander verbunden sind, wobei der Verbindungsabschnitt einer Clipbranche einen in Richtung der anderen Clipbranche hervorstehenden Bolzen aufweist und der Bolzen in eine Ausnehmung der anderen Clipbranche eingreift und dadurch eine scharnierartige drehbare Verbindung zwischen den Verbindungsabschnitten der beiden Clipbranchen ausgebildet ist, wobei der zusätzliche Drehpunkt bzw. die zusätzliche Drehachse durch den Bolzen ausgebildet ist.

Der gemeinsame zusätzliche Drehpunkt der beiden Clipbranchen ist somit erfindungsgemäß insbesondere durch eine Bolzenverbindung ausgebildet. Indem der Verbindungsabschnitt von einer Clipbranche einen hervorstehenden Bolzen aufweist, welcher in eine Ausnehmung in dem Verbindungsabschnitt der zweiten Clipbranche eingreifend ist, wird der zusätzliche gemeinsame Drehpunkt in einfacher Weise ausgebildet. Erfindungsgemäß kann es jedoch auch vorgesehen sein, den Bolzen als separates Bauteil auszuführen, welcher dann wiederum in jeweils in den Verbindungsabschnitten der beiden Clipbranchen vorgesehenen Ausnehmungen eingreift.

Ein vorteilhaftes Ausführungsbeispiel ist dabei dadurch gekennzeichnet, dass der Bolzen einen rotatorischen und einen translatorischen Freiheitsgrad aufweist und die Federwegbegrenzungseinrichtung durch eine Begrenzung der translatorischen Bewegung des Bolzens ausgebildet ist.

Aufgrund des translatorischen Freiheitsgrads des Bolzens, insbesondere in Krafteinleitungsrichtung, erfolgt somit bei einer Krafteinleitung zunächst eine Auslenkung der beiden Clipbranchen um das erste Federelement bzw. die Schenkelfeder. Anschließend erfolgt eine Begrenzung der translatorischen Bewegung durch die erfindungsgemäße Federwegbegrenzungseinrichtung bzw. den Anschlag. Somit wird die Auslenkung der beiden Clipbranchen um den Federdrehpunkt der Schenkelfeder begrenzt. Bei einer weiteren Kraftaufbringung erfolgen schließlich eine Auslenkung/ Verbiegung der zweiten/ dritten Federelemente, insbesondere der vorgesehenen Blattfedern oder Biegefedern, und eine damit einhergehende Drehung der Clipbranchen um ihren gemeinsamen zusätzlichen Drehpunkt. Dadurch wird realisiert, dass bei einer Kraftaufbringung auf die Clipbranchen zunächst eine Drehung der Branchen um den Federdrehpunkt erfolgt und erst später bzw. anschließend eine Drehung um den zusätzlichen gemeinsamen Drehpunkt. Somit erfolgt ein Umschalten auf den gemeinsamen zusätzlichen Drehpunkt und eine Auslenkung der zweiten und dritten Federelemente, bevor die Kraft zur Überwindung der Schließkraft des ersten Federelements zu hoch wird. Das Umschalten auf den gemeinsamen zusätzlichen Drehpunkt hat eine in geringerem Maße ansteigende Kraft sowie eine übersetzte Öffnungsweite zur Folge. Dadurch, dass zunächst lediglich eine translatorische Bewegung des Bolzens und keine rotatorische Bewegung, i.e. auch keine Auslenkung der zweiten und dritten Federelemente, erfolgt, wird sichergestellt, dass bei einer Schließstellung des Clips die Schließkraft nur durch das erste Federelement aufgebracht wird und somit keine negative Beeinflussung durch die zweiten/ dritten Federelemente im Hinblick auf die Schließkraft vorliegt.

Dabei ist es zweckmäßig, wenn die Ausnehmung der anderen Clipbranche nach Art eines Langlochs ausgebildet ist, wobei der Bolzen in einer Ruhestellung des chirurgischen Clips an einer abgerundeten Seite des Langlochs angeordnet ist, durch die Druckkrafteinleitung zum Öffnen des chirurgischen Clips in dem Langloch translatorisch bewegbar ist und die Federwegbegrenzungseinrichtung durch ein Anlegen des Bolzens an die andere abgerundete Seite des Langlochs ausgebildet ist.

Durch die Ausbildung der Ausnehmung als ein Langloch kann in einfacher Weise die translatorische Bewegung des Bolzens ermöglicht werden. Der Anschlag zur Begrenzung der translatorischen Bewegung erfolgt nach dieser Ausführungsform durch Anlage des Bolzens an einer abgerundeten Seite des Langlochs. Durch die Anlage des Bolzens an der abgerundeten Seite werden zudem die anschließende Drehbewegung des Bolzens und das damit einhergehende weitere Öffnen der Clipbranchen ermöglicht. Somit stellt eine Ausbildung der Ausnehmung als ein Langloch in einfacher Weise sowohl einen translatorischen als auch einen rotatorischen Freiheitsgrad eines darin aufgenommenen Bolzens zur Verfügung.

In vorteilhafter Weise erfolgt erfindungsgemäß somit bei der Druckkrafteinleitung zum Öffnen des chirurgischen Clips zunächst hauptsächlich eine translatorische Bewegung des Bolzens in der vorgesehenen Ausnehmung und die beiden Clipbranchen sind um den von dem ersten Federelement ausgebildeten Federdrehpunkt auslenkbar, wobei nach der Begrenzung der translatorischen Bewegung durch die Federwegbegrenzungseinrichtung die rotatorische Bewegung der beiden Clipbranchen um den von dem Bolzen ausgebildeten zusätzlichen Drehpunkt bei einer gleichzeitigen Auslenkung/ Verbiegung der zweiten und dritten Federelemente erfolgt.

Gemäß einem anderen Aspekt ist es erfindungsgemäß außerdem zweckmäßig, wenn die zweiten und dritten Federelemente integral und/oder einmaterialig mit den beiden Clipbranchen und vorzugsweise durch eine geeignete konstruktive Dimensionierung der Federabschnitte der beiden Clipbranchen ausgebildet sind.

Durch die integrale und/oder einmaterialige Ausbildung der zweiten und dritten Federelemente mit den Federabschnitten wird erreicht, dass keine nachträgliche Montage der zweiten und dritten Federelemente vonnöten ist. Die integrale bzw. einmaterialige Ausbildung der zweiten und dritten Federelemente mit dem Federabschnitt wird dabei insbesondere dadurch erreicht, dass an den Überkreuzungsabschnitten, an welchen sich die Clipbranchen jeweils in den Federabschnitt und den Verbindungsabschnitt aufspalten, die Federabschnitte sehr dünn, das heißt mit einer geringen Materialtiefe bzw. mit einem geringeren Querschnitt ausgebildet sind und dadurch die Federelemente, insbesondere nach Art einer Blattfeder oder Biegefeder, ausgebildet werden und die Federabschnitte zu dem Krafteinleitungspfad hin bis zu dem ersten Federelement wiederum um einiges dicker mit einer größeren Materialtiefe bzw. mit einem größeren Querschnitt ausgebildet sind, so dass ab einer vorbestimmten Kraft eine Auslenkung/ Verbiegung der dünner ausgebildeten Abschnitte der Federabschnitte erfolgen kann.

Außerdem betrifft die vorliegende Offenbarung ein Verfahren zum Öffnen eines chirurgischen Clips, insbesondere eines Aneurysmen-Clips, insbesondere eines Clips wie voranstehend beschrieben, wobei der chirurgische Clip zwei Clipbranchen aufweist, welche an ihren proximalen Enden über ein erstes Federelement, insbesondere eine Schenkelfeder, federelastisch verbunden und vorgespannt sind, welche um einen von dem ersten Federelement ausgebildeten Federdrehpunkt drehbar sind, welche an ihren distalen, freien Enden in einer Ruhestellung des chirurgischen Clips durch die Schließkraft des ersten Federelements parallel aneinander anliegend gehalten sind, und welche Überkreuzungsabschnitte zwischen ihren proximalen und distalen Enden aufweisen, an welchen sich die Clipbranchen gegenseitig überkreuzen, gekennzeichnet durch die Schritte: Aufbringen einer Druckkraft auf die beiden Clipbranchen auf einem zwischen den Überkreuzungsabschnitten und dem ersten Federelement ausgebildeten Krafteinleitungspfad und dadurch Auslenkung der beiden Clipbranchen um den Federdrehpunkt; Begrenzung der Auslenkung um den Federdrehpunkt durch eine Federwegbegrenzungseinrichtung, insbesondere einen Anschlag; Weiteres Aufbringen einer Druckkraft auf die beiden Clipbranchen und damit einhergehendes Auslenken von zumindest einer Clipbranche um einen zusätzlich vorgesehenen Drehpunkt, welcher distal von dem Federdrehpunkt und proximal von dem Krafteinleitungspfad angeordnet ist, unter Verbiegung/ Überwindung einer federelastischen Vorspannung zumindest eines zweiten Federelements, insbesondere einer Blattfeder oder einer Biegefeder.

Mit anderen Worten betrifft die Erfindung einen übersetzten Aneurysmen-Clip. Durch den Einsatz von zumindest zwei Federn (einer Schenkelfeder und einer Biegefeder/ Blattfeder) kann eine Segmentierung bzw. Trennung der Arbeitsbereiche der Federn erfolgen, so dass eine Öffnungsweite des Aneurysmen-Clips vergrößert werden kann, ohne die Grenzen der Schenkelfeder bzw. Primärfeder zu überschreiten. Durch einen Wechsel des Drehpunkts in dem Clipbranchen-/ bzw. Maulteilbranchensystem kann bei einer geringeren Winkelbewegung der Schenkelfeder eine übersetzte Drehung der Clipbranchen bei geringer Biegung der Biegefeder/ Blattfeder erreicht werden. Erfindungsgemäß wird somit bei einer großen Schließkraft eine größere Öffnungsweite bzw. ein größerer Arbeitsbereich der Clipbranchen erreicht.

Die Erfindung wird nachfolgend mit Hilfe von Figuren weiter erläutert. Es zeigen:
- Fig. 1: eine Längsansicht einer Ausführungsform eines erfindungsgemäßen chirurgischen Clips in einer Schließstellung der Clipbranchen;
- Fig. 2: eine Längsansicht des Clips der Fig. 1 in einem leicht geöffneten Zustand kurz vor einem Wechsel des Drehpunkts;
- Fig. 3: eine Längsansicht des Clips der Fig. 1 und Fig. 2 in einem weit geöffneten Zustand nach einem Wechsel des Drehpunkts;
- Fig. 4: eine schematische Ansicht, welche die Auswirkungen des erfindungsgemäßen Drehpunkwechsels auf die Öffnungsweite der beiden Clipbranchen verdeutlicht;
- Fig. 5: ein Diagramm, welches einen erfindungsgemäßen Zusammenhang zwischen einem Branchenöffnungswinkel an den distalen Enden der Clipbranchen und einer resultierenden aufzubringenden Kraft verdeutlicht; und
- Fig. 6: ein Diagramm, welches einen erfindungsgemäßen Zusammenhang zwischen einem Auslenkungswinkel an dem Krafteinleitungspfad und einem resultierenden Branchenöffnungswinkel an den distalen Enden der Clipbranchen verdeutlicht.

Die Figuren sind lediglich schematischer Natur und dienen ausschließlich dem Verständnis der Erfindung. Gleiche Elemente sind dabei mit denselben Bezugszeichen versehen. Die Merkmale der einzelnen Ausführungsbeispiele können untereinander ausgetauscht werden.

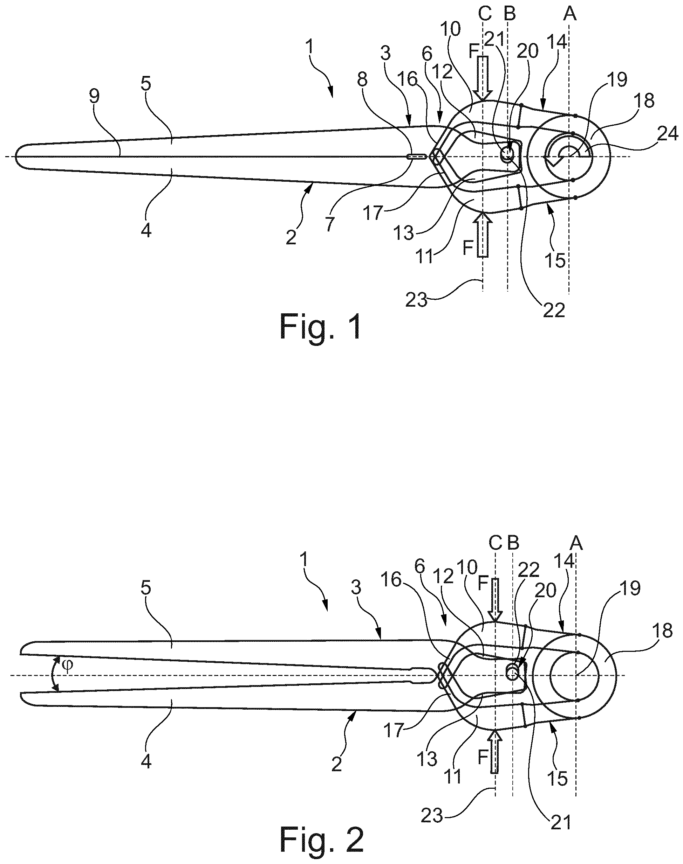

In Fig. 1 ist ein Aneurysmen-Clip 1 gezeigt. Der Aneurysmen-Clip 1 enthält eine erste Clipbranche 2 und eine zweite Clipbranche 3. Die Clipbranchen 2, 3 weisen jeweils freie, distale Enden 4, 5 auf, welche in der in Fig. 1 dargestellten Schließstellung des Aneurysmen-Clips 1 parallel aneinander anliegend gehalten sind und eine geeignet große Schließkraft zum Abklemmen eines Aneurysmas aufeinander aufbringen. Die beiden Clipbranchen 2, 3 überkreuzen sich gegenseitig an einem Überkreuzungsbereich 6, wobei die Clipbranchen 2, 3 bis kurz vor dem Überkreuzungsbereich 6 in der Schließstellung parallel aneinander anliegend sind. Kurz vor dem Überkreuzungsbereich 6 weisen die Clipbranchen 2, 3 Ausnehmungen 7, 8 auf, welche eine Anlagefläche 9 der Clipbranchen 2, 3 in der Schließstellung in proximale Richtung hin begrenzen.

An dem Überkreuzungsbereich 6 spalten sich die beiden Clipbranchen 2, 3 in außen liegende Abschnitte bzw. Federabschnitte 10, 11 und innenliegende Abschnitte bzw. Verbindungsabschnitte 12, 13 auf. Der Federabschnitt 10 und der Verbindungsabschnitt 12 bilden dabei zusammen das proximale Ende 14 der ersten Clipbranche 2. Der Federabschnitt 11 und der Verbindungsabschnitt 13 bilden zusammen das proximale Ende 15 der zweiten Clipbranche 3. Die außen liegenden Abschnitte bzw. Federabschnitte 10, 11 sind in dem Überkreuzungsbereich 6 sehr dünn ausgebildet und bilden dadurch Blattfedern 16, 17 aus.

Nach dem Überkreuzungsbereich 6 in proximale Richtung hin sind die außen liegenden Federabschnitte 10, 11 dicker, das heißt mit einem größeren Querschnitt ausgebildet und schließlich mit einer Schenkelfeder 18 verbunden. Die Schenkelfeder 18 bildet dabei einen Schenkelfederdrehpunkt 19 aus, um welchen die beiden Clipbranchen 2, 3 grundsätzlich drehbar sind. In der Schließstellung des Aneurysmen-Clips 1 wird die Schließkraft durch die Schenkelfeder 18 auf die distalen Enden 4, 5 der Clipbranchen 2, 3 aufgebracht. Die Verbindungsabschnitte 12, 13 der beiden Clipbranchen 2, 3 sind in dem Überkreuzungsbereich 6 wesentlich dicker, das heißt mit einem größeren Querschnitt, als die Federabschnitte 10, 11 ausgebildet.

Die Verbindungsabschnitte 12, 13 sind über eine Bolzenverbindung 20 miteinander verbunden. Die Bolzenverbindung 20 ist dabei derart ausgebildet, dass der Verbindungsabschnitt 12 der Clipbranche 2 einen Bolzen 21 aufweist, welcher in eine in dem Verbindungsabschnitt 13 der Clipbranche 3 vorgesehenen Langlochausnehmung 22 eingreift. Der Bolzen 21 ist dabei in der Ruhestellung bzw. Schließstellung des Aneurysmen-Clips 1 an einem, in Fig. 1 oberen, Ende der Langlochausnehmung 22 angeordnet. Eine Kraft F zum Öffnen des Aneurysmen-Clips wird an einem Krafteinleitungspfad 23 auf die Federabschnitte 10, 11 der beiden Clipbranchen 2, 3 aufgebracht. Der Krafteinleitungspfad 23 wird dabei durch die größte Erstreckung des Aneurysmen-Clips 1 in Höhenrichtung vorgegeben. Wird jeweils eine Kraft F gegeneinander auf die Federabschnitte 10, 11 in der Konfiguration der Fig. 1 aufgebracht, hat dies zunächst eine Drehung der Clipbranchen 2, 3 um den Schenkelfederdrehpunkt 19 mit der in Fig. 1 angegebenen Drehrichtung 24 zur Folge. Die Bolzenverbindung 20 ist proximal von dem Krafteinleitungspfad 23 und distal von der Schenkelfeder 18, das heißt zwischen dem Krafteinleitungspfad 23 und der Schenkelfeder 18 angeordnet. Der Aneurysmen-Clip 1 ist aus Metall, beispielsweise aus Titan, ausgebildet. Ferner ist der Aneurysmen-Clip 1 als ein einstückiges bzw. einmaterialiges bzw. integrales Bauteil ausgebildet.

Fig. 2 zeigt den Aneurysmen-Clip 1 der Fig.1 in einem leicht geöffneten Zustand unmittelbar vor einem Drehpunktwechsel. Durch Aufbringen der Kraft F an dem Krafteinleitungspfad 23 wurden die Clipbranchen 2, 3 um den Schenkelfederdrehpunkt 19 ausgelenkt, so dass die distalen Enden 4, 5 einen Branchenöffnungswinkel ϕ ausbilden. Der Branchenöffnungswinkel ϕ liegt dabei in der in Fig. 2 gezeigten Konfiguration in einem Bereich zwischen 3° und 10° und entspricht dem erfindungsgemäßen vorbestimmten Branchenöffnungswinkel ϕ_{vorbestimmt}. Eine weitere Auslenkung der Clipbranchen 2, 3 um den Schenkelfederdrehpunkt 19 wird dadurch verhindert, dass der Bolzen 21 der Bolzenverbindung 20 nun an dem anderen, in Fig. 2 unteren, Ende der Langlochausnehmung 22 angeordnet ist. Eine weitere Auslenkung der distalen Enden 4, 5 der Clipbranchen 2, 3 um den Schenkelfederdrehpunkt 19 wird dadurch blockiert. Wird nun ausgehend von dem in Fig. 2 gezeigten Zustand weiterhin eine Kraft F auf die Federabschnitte 10, 11 aufgebracht, wird die Kraft F vorzugsweise unmittelbar nach der Blockierung der translatorischen Bewegung des Bolzens 21 groß genug, um die Blattfedern 16, 17 anfänglich auszulenken bzw. zu verbiegen. Eine sehr geringe weitere Auslenkung der Federabschnitte 10, 11 um den Schenkelfederdrehpunkt 19 hat dabei vorzugsweise die Auslenkung bzw. Biegung der Blattfedern 16, 17 zur Folge. Dadurch, dass die Blattfedern 16, 17 gebogen werden und dadurch, dass der Krafteinleitungspfad 23 distal von der Bolzenverbindung 20 angeordnet ist, wird nun eine Drehung der distalen Enden 4, 5 der Clipbranchen 2, 3, jeweils zusammen mit den Verbindungsabschnitten 12, 13 um den Bolzen 21 ermöglicht. Es findet somit in der in Fig. 2 gezeigten Konfiguration ein Wechsel des Drehpunkts von A nach B bzw. von dem Schenkelfederdrehpunkt 19 zu dem Bolzen 21 statt.

Aus dieser Beschreibung wird klar, dass die Ausbildungen bzw. konstruktiven Gestaltungen bzw. mechanischen Eigenschaften der Blattfedern 16, 17, der Schenkelfeder 18 sowie der Langlochausnehmung 22 präzise aufeinander abgestimmt sein müssen. Insbesondere soll dabei vermieden werden, dass sich die Blattfedern 16, 17 schon während der translatorischen Bewegung des Bolzens 21 in der Langlochausnehmung 22 verbiegen. Außerdem sollen die Blattfedern 16, 17 derart ausgebildet sein, dass bei Beginn des Arbeitsbereichs der Blattfedern 16, 17 eine resultierende aufzubringende Kraft an dem Krafteinleitungspfad 23 mit zunehmendem Auslenkungswinkel in geringerem Maße ansteigend ist als in dem Arbeitsbereich der Schenkelfeder 18. Erfindungsgemäß ist somit eine klare Trennung zwischen dem Arbeitsbereich der Schenkelfeder 18 und dem Arbeitsbereich der Blattfedern 16, 17 vorgesehen.

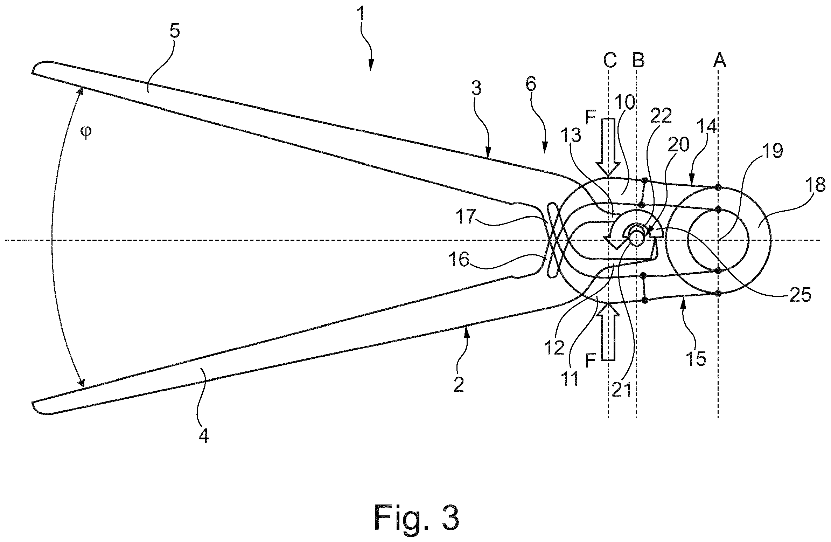

Fig. 3 zeigt den Aneurysmen-Clip 1 der Fig.1 und Fig. 2 in einem weit geöffneten Zustand nach einem Wechsel des Drehpunkts von A nach B. Wird ausgehend von dem in Fig. 2 gezeigten Zustand weiter eine Kraft auf dem Krafteinleitungspfad 23 bei C aufgebracht, verbiegen sich die Blattfedern 16, 17 und der Verbindungsabschnitt 12 dreht sich zusammen mit dem distalen Ende 4 um den von dem Bolzen 21 ausgebildeten Drehpunkt bei B in der in Fig. 3 angegebenen Drehrichtung 25. Der Verbindungsabschnitt 13 und das distale Ende 5 drehen sich entsprechend entgegen der in Fig. 3 angegebenen Drehrichtung 25. Durch den Drehpunktwechsel von A nach B wird dabei eine stark übersetzte Drehung der distalen Enden 4, 5 der Clipbranchen 2, 3 erreicht, so dass nun ein großer Branchenöffnungswinkel ϕ erreicht wird. Der Branchenöffnungswinkel ϕ beträgt in Fig. 3 etwa 30° bis 35°. Werden nun ausgehend von der Konfiguration der Fig. 3 die distalen Enden 4, 5 der Clipbranchen 2, 3 wieder geschlossen, entspannen sich zunächst die Blattfedern 16, 17 wieder und gehen in ihren unverbogenen Zustand zurück und man gelangt wieder zu der Konfiguration der Fig. 2. Ausgehend davon erfolgt nun beim Schließen wiederum eine translatorische Bewegung des Bolzens 21 in der Langlochausnehmung 22 und die distalen Enden 4, 5 kommen wiederum aufeinander zum Anliegen, wobei die Schließkraft lediglich von der Schenkelfeder 18 auf die Clipbranchen 2, 3 aufgebracht wird. Somit beeinflussen die Blattfedern 16, 17 die Schließkraft des Aneurysmen-Clips 1 in keinerlei Weise.

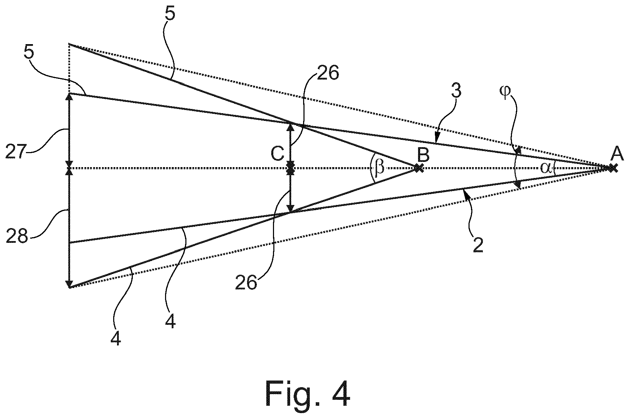

Fig. 4 zeigt eine schematische Ansicht, welche die Auswirkungen des erfindungsgemäßen Drehpunkwechsels auf die Öffnungsweite/ den Branchenöffnungswinkel ϕ der beiden Clipbranchen 2, 3 verdeutlicht. Dabei wird auf die Clipbranchen 2, 3 bei C eine Kraft aufgebracht wird, welche wiederum eine Auslenkung 26 bei C zur Folge hat. Erfindungsgemäß wird dadurch ein Winkel α (Öffnungswinkel bei A) zwischen den beiden Clipbranchen 2, 3 eingeschlossen. Erreicht der Winkel α den voranstehend beschriebenen Branchenöffnungswinkel ϕ_{vorbestimmt}, liegt eine erste Branchenöffnungsweite 27 vor und eine Drehung der Clipbranchen 2, 3 um den Punkt A wird blockiert. Dafür ist beispielsweise in einem Bereich 0° ≤ ϕ ≤ 10° eine Kraft F = k_{A} × α vonnöten, wobei k_{A} die Federkonstante der (Schenkel-) Feder bei A bezeichnet. Bei weiterer Kraftaufbringung bei C erfolgt ein Drehpunktwechsel von A auf B. Nun öffnen sich die distalen Enden 4, 5 der Clipbranchen 2, 3 weiter und schließen bei dem neuen Drehpunkt B einen Winkel β (Öffnungswinkel bei B) bei einer zweiten Branchenöffnungsweite 28 ein. Beispielsweise findet der Drehpunktwechsel bei ϕ = 10° statt und es ist für einen Bereich ϕ > 10° eine Kraft F = k_{A} × α + k_{B} × β vonnöten, wobei k_{B} die Federkonstante der Blatt- bzw. Biegefeder(n), welche für eine weitere Auslenkung verbogen werden, bezeichnet. Dabei ist k_{B} vorzugsweise geringer als k_{A}. Ein Wechsel des Drehpunkts von A nach B, wie dies gemäß der vorliegenden Erfindung vorgesehen ist, führt somit vorzugsweise bei einer geringeren Kraftaufbringung - im Vergleich zu einer Drehung der Clipbranchen 2, 3 lediglich um A - zu einer größeren erreichbaren Branchenöffnungsweite ϕ an den distalen Enden 4, 5.

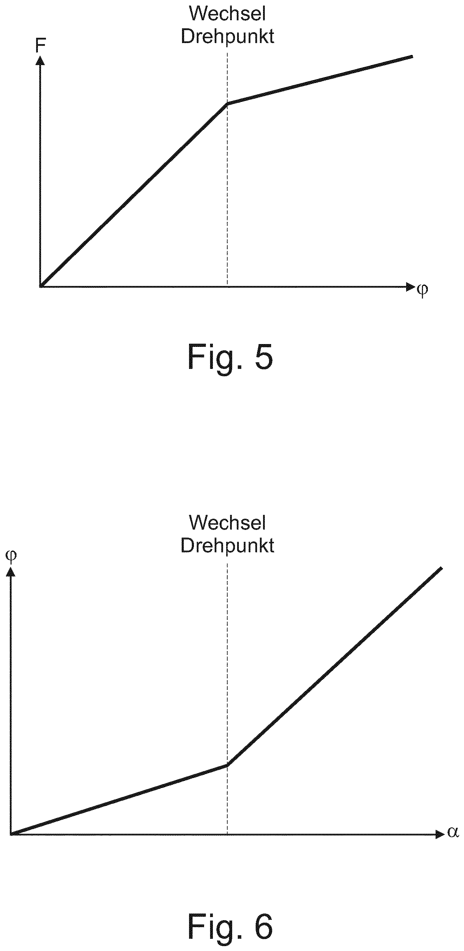

Fig. 5 zeigt ein Diagramm, welches einen erfindungsgemäßen Zusammenhang zwischen einem Branchenöffnungswinkel ϕ an den distalen Enden 4, 5 der Clipbranchen 2, 3 und einer resultierenden aufzubringenden Kraft verdeutlicht. In einem Arbeitsbereich der Schenkelfeder 18 steigt dabei zunächst die aufzubringende Kraft mit zunehmendem Branchenöffnungswinkel ϕ linear und kontinuierlich an. Die resultierende aufzubringende Kraft und der Auslenkungswinkel sind somit proportional zueinander. Ab dem Drehpunktwechsel von A nach B und somit mit Beginn des Arbeitsbereichs der Blattfedern 16, 17 steigt die aufzubringende Kraft mit zunehmendem Branchenöffnungswinkel ϕ in geringerem Maße linear an.

Fig. 6 zeigt ein Diagramm, welches einen erfindungsgemäßen Zusammenhang zwischen einem Auslenkungswinkel an dem Krafteinleitungspfad bzw. einem Öffnungswinkel bei A (Winkel α) und einem resultierenden Öffnungswinkel der Clipbranchen ϕ verdeutlicht. Hier steigt zunächst in dem Arbeitsbereich der Schenkelfeder 18 der resultierende Clipbranchen-Öffnungswinkel ϕ mit zunehmendem Auslenkungswinkel α an dem Krafteinleitungspfad 23 in geringem Maße an. Aufgrund des Drehpunktwechsels von A nach B steigt in dem Arbeitsbereich der Blattfedern 16, 17 der resultierende Clipbranchen-Öffnungswinkel ϕ aufgrund der übersetzten Drehung in weitaus stärkerem Maße linear an.

### Bezugszeichenliste

- 1: Aneurysmen-Clip
- 2: erste Clipbranche
- 3: zweite Clipbranche
- 4: distales Ende (erste Clipbranche)
- 5: distales Ende (zweite Clipbranche)
- 6: Überkreuzungsbereich
- 7: Ausnehmung (erste Clipbranche)
- 8: Ausnehmung (zweite Clipbranche)
- 9: Anlagefläche
- 10: Federabschnitt (erste Clipbranche)
- 11: Federabschnitt (zweite Clipbranche)
- 12: Verbindungsabschnitt (erste Clipbranche)
- 13: Verbindungsabschnitt (zweite Clipbranche)
- 14: proximales Ende (erste Clipbranche)
- 15: proximales Ende (zweite Clipbranche)
- 16: Blattfeder (erste Clipbranche)
- 17: Blattfeder (zweite Clipbranche)
- 18: Schenkelfeder
- 19: Schenkelfederdrehpunkt
- 20: Bolzenverbindung
- 21: Bolzen
- 22: Langlochausnehmung
- 23: Krafteinleitungspfad
- 24: Drehrichtung (Schenkeldrehpunkt)
- 25: Drehrichtung (Bolzen)
- 26: Auslenkung
- 27: erste Branchenöffnungsweite
- 28: zweite Branchenöffnungsweite

## Patentansprüche

1. Chirurgischer Clip (1), insbesondere Aneurysmen-Clip, mit zwei Clipbranchen (2, 3), welche an ihren proximalen Enden (14, 15) über ein erstes Federelement (18), insbesondere eine Schenkelfeder, federelastisch verbunden und vorgespannt sind, welche um einen von dem ersten Federelement (18) ausgebildeten Federdrehpunkt (19) drehbar sind und welche an ihren distalen, freien Enden (4, 5) in einer Ruhestellung des chirurgischen Clips (1) durch die Schließkraft des ersten Federelements (18) parallel aneinander anliegend gehalten sind, **dadurch gekennzeichnet, dass** der chirurgische Clip (1) eine Übersetzungseinrichtung aufweist, welche ab einem vorbestimmten Öffnungswinkel (ϕ_{vorbestimmt}) der distalen, freien Enden (4, 5) der beiden Clipbranchen (2, 3) ein Verhältnis von Öffnungskraft (F), welche zum Öffnen der Clipbranchen (2, 3) an einem Krafteinleitungspfad (23) aufgebracht wird, zu Öffnungswinkel (ϕ) verringert, wobei zumindest eine der Clipbranchen (2, 3) einen zusätzlichen Drehpunkt aufweist, welcher distal zu dem Federdrehpunkt (19) und proximal zu dem Krafteinleitungspfad (23) angeordnet ist und um welchen die zumindest eine Clipbranche (2, 3) unter Überwindung einer federelastischen Vorspannung zumindest eines zweiten Federelements (16, 17), insbesondere einer Blattfeder oder einer Biegefeder, drehbar ist.

2. Chirurgischer Clip (1) nach Anspruch 1, **dadurch gekennzeichnet, dass** die beiden Clipbranchen (2, 3) jeweils einen zusätzlichen Drehpunkt aufweisen, um welche die beiden Clipbranchen (2, 3) jeweils unter Überwindung einer federelastischen Vorspannung eines zweiten und eines dritten Federelements (16, 17), welche insbesondere als eine Blattfeder oder eine Biegefeder ausgebildet sind, drehbar sind, wobei die beiden zusätzlichen Drehpunkte vorzugsweise von dem ersten Federelement (18) distal und von dem Krafteinleitungspfad (23) proximal gleich beabstandet sind.

3. Chirurgischer Clip (1) nach Anspruch 2, **gekennzeichnet ferner durch** eine Federwegbegrenzungseinrichtung, insbesondere einen Anschlag, welche eine Auslenkung (26) und/oder einen Öffnungswinkel (27) der beiden Clipbranchen (2, 3) um den von dem ersten Federelement (18) ausgebildeten Federdrehpunkt (19) begrenzt.

4. Chirurgischer Clip (1) nach Anspruch 2 oder 3, **dadurch gekennzeichnet, dass** die zwei Clipbranchen (2, 3) an Überkreuzungsabschnitten (6), an welchen sich die Clipbranchen (2, 3) gegenseitig überkreuzen, jeweils einen Federabschnitt (10, 11) und einen Verbindungsabschnitt (12, 13) aufweisen, wobei der Federabschnitt (10) der einen Clipbranche (2) das zweite Federelement (16) und der Federabschnitt (11) der anderen Clipbranche (3) das dritte Federelement (17) aufweisen und wobei die Federabschnitte (10, 11) jeweils mit dem ersten Federelement (18) verbunden sind, wobei die Verbindungsabschnitte (12, 13) der zwei Clipbranchen (2, 3) derart drehbar miteinander verbunden sind, dass die zusätzlichen Drehpunkte der beiden Clipbranchen (2, 3) als ein gemeinsamer zusätzlicher Drehpunkt ausgebildet sind und die Clipbranchen (2, 3) jeweils um den gemeinsamen zusätzlichen Drehpunkt über eine Auslenkung und/oder Verbiegung und/oder Überwindung der federelastischen Vorspannung der zweiten und dritten Federelemente (16, 17) drehbar sind.

5. Chirurgischer Clip (1) nach Anspruch 4, **dadurch gekennzeichnet, dass** die Verbindungsabschnitte (12, 13) der beiden Clipbranchen (2, 3) über eine Bolzenverbindung (20) drehbar miteinander verbunden sind, wobei der Verbindungsabschnitt (12) einer Clipbranche (2) einen in Richtung der anderen Clipbranche (3) hervorstehenden Bolzen (21) aufweist und der Bolzen (21) in eine Ausnehmung (22) der anderen Clipbranche (3) eingreifend ist und dadurch eine scharnierartige drehbare Verbindung zwischen den Verbindungsabschnitten (12, 13) der beiden Clipbranchen ausgebildet ist, wobei der zusätzliche Drehpunkt durch den Bolzen (21) ausgebildet ist.

6. Chirurgischer Clip (1) nach Anspruch 5, **dadurch gekennzeichnet, dass** der Bolzen (21) einen rotatorischen und einen translatorischen Freiheitsgrad aufweist und die Federwegbegrenzungseinrichtung durch eine Begrenzung der translatorischen Bewegung des Bolzens (21) ausgebildet ist.

7. Chirurgischer Clip (1) nach Anspruch 5 oder 6, **dadurch gekennzeichnet, dass** die Ausnehmung (22) der anderen Clipbranche (3) nach Art eines Langlochs ausgebildet ist, wobei der Bolzen (21) in einer Ruhestellung des chirurgischen Clips (1) an einer abgerundeten Seite des Langlochs angeordnet ist, durch die Druckkrafteinleitung zum Öffnen des chirurgischen Clips (1) in dem Langloch translatorisch bewegbar ist und die Federwegbegrenzungseinrichtung durch ein Anlegen des Bolzens an die andere abgerundete Seite des Langlochs ausgebildet ist.

8. Chirurgischer Clip (1) nach einem der Ansprüche 3 bis 7, **dadurch gekennzeichnet, dass** die zweiten und dritten Federelemente (16,17) integral und/oder einmaterialig mit den beiden Clipbranchen (2, 3), insbesondere durch eine geeignete konstruktive Dimensionierung der Federabschnitte (10, 11) der beiden Clipbranchen (2, 3) ausgebildet sind.

## Claims

1. A surgical clip (1), in particular an aneurism clip, comprising two clip branches (2, 3) which are spring-elastically joined and pretensioned at the proximal ends (14, 15) thereof via a first spring element (18), in particular a leg spring, which can be rotated about a spring pivot point (19) formed by the first spring element (18), and which are held with the distal free ends (4, 5) thereof in parallel against one another in a resting position of the surgical clip (1) via the closing force of the first spring element (18), **characterized in that** the surgical clip (1) has a conversion device which reduces a ratio of opening force (F), applied to a force-guiding path (23) for opening the clip branches (2, 3), to opening angle (ϕ), from a predetermined opening angle (ϕ_{predetermined}) of the distal free ends (4, 5) of the two clip branches (2, 3), wherein at least either of the clip branches (2, 3) has an additional pivot point which is arranged distally from the spring pivot point (19) and proximally from the force-guiding path (23) and about which the at least one clip branch (2, 3) can be rotated while overcoming a spring-elastic pretension at least of a second spring element (16, 17), especially a leaf spring or a flexible spring.

2. The surgical clip (1) according to claim 1, **characterized in that** each of the two clip branches (2, 3) has an additional pivot point about which the two clip branches (2, 3) can be rotated while overcoming a spring-elastic pretension of second and third spring elements (16, 17) which are especially in the form of a leaf spring or a flexible spring, wherein the two additional pivot points are preferably equally spaced apart distally from the first spring element (18) and proximally from the force-guiding path (23).

3. The surgical clip (1) according to claim 2, **further characterized by** a spring travel limiting unit, in particular a stop, which limits a deflection (26) and/or an opening angle (27) of the two clip branches (2, 3) about the spring pivot point (19) formed by the first spring element (18).

4. The surgical clip (1) according to claim 2 or 3, **characterized in that** the two clip branches (2, 3) each include a spring portion (10, 11) and a connecting portion (12, 13) at crossing portions (6) where the clip branches (2, 3) are mutually crossing, wherein the spring portion (10) of the one clip branch (2) includes the second spring element (16), and the spring portion (11) of the other clip branch (3) includes the third spring element (17), and wherein each of the spring portions (10, 11) is connected to the first spring element (18), the connecting portions (12, 13) of the two clip branches (2, 3) being rotatably connected to each other such that the additional pivot points of the two clip branches (2, 3) are in the form of a joint additional pivot point and each of the clip branches (2, 3) can be rotated about the joint additional pivot point via deflection and/or bending and/or overcoming of the spring-elastic pretension of the second and third spring elements (16, 17).

5. The surgical clip (1) according to claim 4, **characterized in that** the connecting portions (12, 13) of the two clip branches (2, 3) are rotatably connected to each other via a pin joint (20), wherein the connecting portion (12) of one clip branch (2) has a pin (21) projecting in the direction of the other clip branch (3) and the pin (21) engages in a recess (22) of the other clip branch (3) and, in this way, a hinge-type rotatable connection is formed between the connecting portions (12, 13) of the two clip branches, with the additional pivot point being formed by the pin (21).

6. The surgical clip (1) according to claim 5, **characterized in that** the pin (21) has a rotational degree of freedom and a translational degree of freedom and the spring travel limiting unit is formed by limitation of the translational movement of the pin (21).

7. The surgical clip (1) according to claim 5 or 6, **characterized in that** the recess (22) of the other clip branch (3) is in the form of a slotted hole, wherein the pin (21) is arranged, in a resting position of the surgical clip (1), at a rounded side of the slotted hole, is translationally movable within the slotted hole by the introduction of pressure for opening the surgical clip (1) and the spring travel limiting unit is formed by applying the pin to the other rounded side of the slotted hole.

8. The surgical clip (1) according to one of the claims 3 to 7, **characterized in that** the second and third spring elements (16, 17) are formed integrally and/or of one single material with the two clip branches (2, 3), in particular by appropriate structural dimensioning of the spring portions (10, 11) of the two clip branches (2, 3).

## Revendications

1. Clamp chirurgical (1), en particulier clamp pour anévrisme, avec deux branches de clamp (2, 3) qui sont reliées et précontraintes de manière élastique au niveau de leurs extrémités proximales (14, 15) par le biais d'un premier élément de ressort (18), en particulier d'un ressort à branches, branches de clamp qui sont rotatives autour d'un point de rotation de ressort (19) réalisé par le premier élément de ressort (18) et qui sont maintenues reposant parallèlement l'une contre l'autre au niveau de leurs extrémités libres distales (4, 5) dans une position de repos du clamp chirurgical (1) par la force de fermeture du premier élément de ressort (18), **caractérisé en ce que** le clamp chirurgical (1) présente un appareil de démultiplication qui diminue à partir d'un angle d'ouverture prédéterminé (ϕ _{prédéterminé}) des extrémités libres distales (4, 5) des deux branches de clamp (2, 3) un rapport entre la force d'ouverture (F) qui est appliquée pour l'ouverture des branches de clamp (2, 3) et une voie d'introduction de force (23) et l'angle d'ouverture (ϕ), dans lequel au moins une des branches de clamp (2, 3) présente un point de rotation supplémentaire qui est agencé de manière distale au point de rotation de ressort (19) et de manière proximale à la voie d'introduction de force (23) et autour duquel l'au moins une branche de clamp (2, 3) est rotative en dépassant une précontrainte élastique au moins d'un second élément de ressort (16, 17), en particulier un ressort à lames ou un ressort spiral.

2. Clamp chirurgical (1) selon la revendication 1, **caractérisé en ce que** les deux branches de clamp (2, 3) présentent respectivement un point de rotation supplémentaire, autour duquel les deux branches de clamp (2, 3) sont rotatives respectivement en dépassant une précontrainte élastique d'un deuxième et d'un troisième élément de ressort (16, 17) qui sont réalisés en particulier comme un ressort à lames ou un ressort spiral, dans lequel les deux points de rotation supplémentaires sont espacés de manière identique de préférence de manière distale du premier élément de ressort (18) et de manière proximale de la voie d'introduction de force (23).

3. Clamp chirurgical (1) selon la revendication 2, **caractérisé de plus par** un appareil de limitation de course de ressort, en particulier une butée, qui limite une déviation (26) et/ou un angle d'ouverture (27) des deux branches de clamp (2, 3) autour du point de rotation de ressort (19) réalisé par le premier élément de ressort (18).

4. Clamp chirurgical (1) selon la revendication 2 ou 3, **caractérisé en ce que** les deux branches de clamp (2, 3) présentent aux sections de croisement (6), au niveau desquelles les branches de clamp (2, 3) se croisent mutuellement, respectivement une section de ressort (10, 11) et une section de liaison (12, 13), dans lequel la section de ressort (10) de l'une branche de clamp (2) présente le deuxième élément de ressort (16) et la section de ressort (11) de l'autre branche de clamp(3) présente le troisième élément de ressort (17) et dans lequel les sections de ressort (10, 11) sont reliées respectivement au premier élément de ressort (18), dans lequel les sections de liaison (12, 13) des deux branches de clamp (2, 3) sont reliées entre elles de manière rotative de telle manière que les points de rotation supplémentaires des deux branches de clamp (2, 3) soient réalisés comme un point de rotation supplémentaire commun et les branches de clamp (2, 3) soient rotatives respectivement autour du point de rotation supplémentaire commun par le biais d'une déviation et/ou d'une déformation et/ou d'un dépassement de la précontrainte élastique des deuxième et troisième élément de ressort (16, 17).

5. Clamp chirurgical (1) selon la revendication 4, **caractérisé en ce que** les sections de liaison (12, 13) des deux branches de clamp (2, 3) sont reliées entre elles de manière rotative par le biais d'une liaison à boulon (20), dans lequel la section de liaison (12) d'une branche de clamp (2) présente un boulon (21) dépassant en direction de l'autre branche de clamp (3) et le boulon (21) est en engagement dans un évidement (22) de l'autre branche de clamp (3) et ainsi une liaison rotative à charnière entre les sections de liaison (12, 13) des deux branches de clamp est réalisée, dans lequel le point de rotation supplémentaire est réalisé par le boulon (21).

6. Clamp chirurgical (1) selon la revendication 5, **caractérisé en ce que** le boulon (21) présente un degré de liberté rotatif et un degré de liberté translatoire et l'appareil de limitation de course de ressort est réalisé par une limitation du mouvement translatoire du boulon (21).

7. Clamp chirurgical (1) selon la revendication 5 ou 6, **caractérisé en ce que** l'évidement (22) de l'autre branche de clamp (3) est réalisé comme un trou oblong, dans lequel le boulon (21) est agencé dans une position de repos du clamp chirurgical (1) au niveau d'un côté arrondi du trou oblong, est mobile de manière translatoire par l'introduction de force de pression pour l'ouverture du clamp chirurgical (1) dans le trou oblong et le dispositif de limitation de course de ressort est réalisé par un placement du boulon contre l'autre côté arrondi du trou oblong.

8. Clamp chirurgical (1) selon l'une quelconque des revendications 3 à 7, **caractérisé en ce que** les deuxième et troisième éléments de ressort (16, 17) sont réalisés d'un seul tenant et/ou en un seul matériau avec les deux branches de clamp (2, 3), en particulier par un dimensionnement constructif approprié des sections de ressort (10, 11) des deux branches de clamp (2, 3).
